# EUROPEAN PATENT APPLICATION

(11) **EP 3 581 971 A1**
(43) Date of publication of application: **18.12.2019**
(21) Application number: 17895776.7
(22) Date of filing: 13.03.2017
(51) Int. Cl.: G01V 3/08, B25J 19/02, G01B 7/00, G01B 7/30, G08G 1/16

(54) **STATE DETERMINATION DEVICE, LEARNING DEVICE, STATE DETERMINATION METHOD, AND PROGRAM**

(30) Priority: 13.02.2017 JP 2017024169
(71) Applicant: Omron Corporation, Kyoto-shi, Kyoto 600-8530 (JP)
(72) Inventor: ANDO, Tanichi, Kizugawa-City, Kyoto 619-0283 (JP)
(74) Representative: Horn Kleimann Waitzhofer Patentanwälte PartG mbB
(86) International application number: PCT/JP2017/010048
(87) International publication number: WO 2018/146825

(57) **Abstract**

Provided is a state determination device that can determine a state of an object in more detail than a case where a change in capacitance between a single pair of electrodes. A state determination device 100 for determining a state of an object that is present in the vicinity of a plurality of electrodes 11 includes: a selection unit 130 configured to select a plurality of electrode pairs from the plurality of electrodes 11; a capacitance pattern measurement unit 150 configured to measure a capacitance pattern for the plurality of electrode pairs; and a neural network that is trained to determine a state of the object based on the capacitance pattern.

## Description

### CROSS-REFERENCES TO RELATED APPLICATIONS

This application claims priority to Japanese Patent Application No. 2017-024169 filed February 13, 2017, the entire contents of which are incorporated herein by reference.

### TECHNICAL FIELD

The present invention relates to a state determination device, a learning device, a state determination method, and a program for the same.

### RELATED ART

Conventionally, a technique is known for determining the presence or approach of an object using a capacitance sensor, which uses the principle that the capacitance of an electrode changes depending on the distance between an object and the electrode. Capacitance sensors may be able to determine even an insulating object, a transparent object, or the like, and thus are used to determine various types of objects.

Patent Document 1 discloses a head rest positioning device that is provided with a sensing electrode for sensing the capacitance between the body of a person seated on a seat and a head rest, and is configured to determine the top position of the head portion and the center position in the horizontal direction, and appropriately adjust the position of the head rest.

### RELATED ART DOCUMENT

### PATENT DOCUMENT

Patent Document 1: JP 2009-50462A

### SUMMARY OF THE INVENTION

### PROBLEM TO BE SOLVED BY THE INVENTION

Capacitance sensors may determine, based on a change in the capacitance between a pair of electrodes, the presence or approach of an object. However, there may be cases where a state of the object cannot be determined in detail only based on a change in the capacitance between the pair of electrodes.

For example, assuming that planar conductors that face each other constitute a pair of electrodes, it is possible to determine whether or not an object is present between the pair of electrodes, or which of the pair of electrodes an object is located close to. However, if an object is present between the pair of electrodes, it is difficult to determine the position of the object with respect to the direction in which the planar conductors extend.

Therefore, according to an aspect of the present invention, it is an object thereof to provide a state determination device and the like that can determine a state of an object in more detail than in a case where a change in the capacitance between a single pair of electrodes is used.

### MEANS FOR SOLVING THE PROBLEMS

According to an aspect of the present invention, a state determination device for determining a state of an object that is present in the vicinity of a plurality of electrodes includes: a selection unit configured to select a plurality of electrode pairs from the plurality of electrodes; a capacitance pattern measurement unit configured to measure a capacitance pattern for the plurality of electrode pairs selected by the selection unit; and a neural network that is trained to determine a state of the object based on the capacitance pattern.

With this aspect, by selecting a plurality of electrode pairs from the plurality of electrodes to measure a capacitance pattern, and determining a state of an object that is present in the vicinity of the plurality of electrodes, it is possible to determine the state of the object in more detail than in a case where a change in the capacitance between a single pair of electrodes is used.

In the above-described aspect, the state determination device may further includes a layout acquisition unit configured to acquire a layout of the plurality of electrodes. Here, the neural network may also determine a state of the object based on, in addition to the capacitance pattern measured by the measurement unit, the layout of the plurality of electrodes acquired by the layout acquisition unit.

With this aspect, by determining a state of the object based on the capacitance pattern and the layout of the plurality of electrodes, the state of the object can be determined in more detail.

In the above-described aspect, the state determination device may further includes: a voltage application unit configured to apply a predetermined voltage to each of the plurality of electrode pairs selected from the plurality of electrodes by the selection unit; and a guard electrode arranged enclosing at least some of the plurality of electrodes. The voltage application unit may apply a reference voltage to one of the electrodes of each of the electrode pairs selected from the plurality of electrodes by the selection unit, and to the guard electrode.

With this aspect, by applying a reference voltage to one of the electrodes of each of the electrode pairs and the guard electrode, an electric field is shielded by the guard electrode, and a capacitance pattern for only one of the two sides of the electrodes can be measured, making it possible to restrict the range for which the state of an object is to be determined.

In the above-described aspect, the state determination device may further includes an environment measurement unit configured to measure environmental data that relates to an environment when a capacitance pattern for the plurality of electrode pairs is measured by the capacitance pattern measurement unit. Here, the neural network may also determine a state of the object based on, in addition to the capacitance pattern measured by the capacitance pattern measurement unit, the environmental data measured by the environment measurement unit.

With this aspect, by determining a state of the object based on the capacitance pattern and the environmental data, the state determination is performed with consideration given to the environment dependence of the capacitance pattern, thus making it possible to reduce an error in the state determination that may be caused by a change in a measurement environment.

In the above-described aspect, the environmental data measured by the environment measurement unit may contain data on at least one of humidity and electromagnetic noise.

With this aspect, state determination is performed with consideration given to the dependence on humidity and electromagnetic noise that may affect the capacitance pattern, thus making it possible to reduce an error that results from a change in humidity and the intensity of electromagnetic noise.

In the above-described aspect, the neural network may determine at least one of presence of the object, a position of the object, an angle of the object, a type of the object, a material of the object, and a distribution of the object.

With this aspect, by determining at least one of presence of the object, a position of the object, an angle of the object, a type of the object, a material of the object, and a distribution of the object, it is possible to determine a state of the object in more detail.

In the above-described aspect, the plurality of electrodes may be provided on a robot hand.

With this aspect, a state of an object to be held or operated by the robot hand can be determined, and more precise operation of the robot hand can be assisted. For example, when holding an object, an operation is possible to uniformly bring a plurality of fingers of the robot hand close to the object.

In the above-described aspect, the plurality of electrodes may be provided on a seat.

With this aspect, it is possible to determine details of the state of a person seated on a seat, such as the physical size and the orientation. For example, by determining the condition of an automobile driver seated on a seat of an automobile, it is possible to determine whether or not the driver is awake.

According to an aspect of the present invention, a learning device for training a neural network may further be provided. The learning device includes a learning control unit configured to perform control such that the neural network is trained using learning data that contains a capacitance pattern for a plurality of electrode pairs selected from a plurality of electrodes.

With this aspect, by performing training using learning data that contains a capacitance pattern for an object, a neural network that can determine a state of the object as a learning result can be obtained, and the state of the object can be determined in more detail than in a case where a change in the capacitance between a single pair of electrodes is used.

In the above-described aspect, the learning data may contain data that relates to a layout of the plurality of electrodes.

With this aspect, by flexibly setting a layout of the plurality of electrodes with respect to an object without using a fixed layout of the plurality of electrodes, it is possible to obtain a learning result of the neural network with which a state of the object can be determined in more detail.

In the above-described aspect, the learning data may contain environmental data that relates to an environment when the capacitance pattern is measured.

With this aspect, the neural network is trained using the learning data that contains a capacitance pattern for an object and environmental data, and even if a measurement environment changes, state determination can be performed with consideration given to the environment dependence of the capacitance pattern, thus making it possible to obtain a learning result with which an error in the state determination can be reduced.

In the above-described aspect, the learning control unit may train the neural network using the learning data and teaching data, and the teaching data may contain an image or a video that shows a state of the object

With this aspect, a user does not need to convert the state of the object into numerical values, and a neural network is trained using an image or a video that objectively shows the state of the object as teaching data, thus making it possible to obtain a more objective and versatile learning result of the neural network.

According to an aspect of the present invention, a state determination method for determining a state of an object that is present in the vicinity of a plurality of electrodes includes: a first step of selecting a plurality of electrode pairs from the plurality of electrodes; a second step of measuring a capacitance pattern for the plurality of electrode pairs selected in the first step; and a third step of determining, with a trained neural network, a state of the object based on the capacitance pattern measured in the second step.

With this aspect, by selecting a plurality of electrode pairs from the plurality of electrodes to measure a capacitance pattern, and determining a state of an object, it is possible to determine the state of the object in more detail than in a case where a change in the capacitance between a single pair of electrodes is used.

According to an aspect of the present invention, a program for state determination causes a computer provided in a state determination device for determining a state of an object that is present in the vicinity of a plurality of electrodes to function as: a selection unit configured to select a plurality of electrode pairs from the plurality of electrodes; a capacitance pattern measurement unit configured to measure a capacitance pattern for the plurality of electrode pairs selected by the selection unit; and a neural network that is trained to determine the state of the object based on the capacitance pattern.

With this aspect, by selecting a plurality of electrode pairs from the plurality of electrodes to measure a capacitance pattern, and determining a state of an object, it is possible to determine the state of the object in more detail than in a case where a change in the capacitance between a single pair of electrodes is used.

### EFFECTS OF THE INVENTION

According to an aspect of the present invention, a state determination device and the like that can determine a state of an object in more detail than in a case where a change in the capacitance between a single pair of electrodes is used.

### BRIEF DESCRIPTION OF THE DRAWINGS

- Fig. 1: is a diagram illustrating an overall concept of a state determination system according to a first embodiment.
- Fig. 2A: is a diagram illustrating an example of a layout of a plurality of electrodes.
- Fig. 2B: is a diagram illustrating an example of the layout of a plurality of electrodes.
- Fig. 3: is a diagram illustrating an example of a specific system configuration of the state determination system according to the first embodiment.
- Fig. 4: is a block diagram illustrating a configuration of the state determination device according to the first embodiment.
- Fig. 5: is a block diagram illustrating a configuration of a learning device according to the first embodiment.
- Fig. 6: is a block diagram illustrating an example of a hardware configuration of constituent devices of the state determination system according to the first embodiment.
- Fig. 7: is a flowchart of learning data generation processing performed by a learning data generation device according to the first embodiment.
- Fig. 8: is a flowchart of learning processing performed by the learning device according to the first embodiment.
- Fig. 9: is a flowchart of object recognition processing performed by the state determination device according to the first embodiment.
- Fig. 10: is a diagram illustrating a situation, in a second embodiment, in which a plurality of electrodes are provided on a robot hand.
- Fig. 11: is a functional block diagram of a state determination device according to the second embodiment.
- Fig. 12: is a diagram illustrating a situation, in a third embodiment, in which a plurality of electrodes are provided on a seat.

### EMBODIMENTS OF THE INVENTION

Embodiments of the present invention will be described with reference to the accompanying drawings. Note that, in the drawings, elements with the same reference numerals have the same or an equivalent configuration. The present specification will describe a first embodiment, and will then describe, as specific working examples, mainly a second embodiment relating to a case where a plurality of electrodes of a state determination device are provided on a robot hand, and a third embodiment relating to a case where the plurality of electrodes of the state determination device are provided on a seat. However, the plurality of electrodes of the state determination device according to the present invention may also be provided on a component other than a robot hand or a seat, and the configuration of the present invention is not limited to the configurations of the embodiments, which will be described below.

### First Embodiment

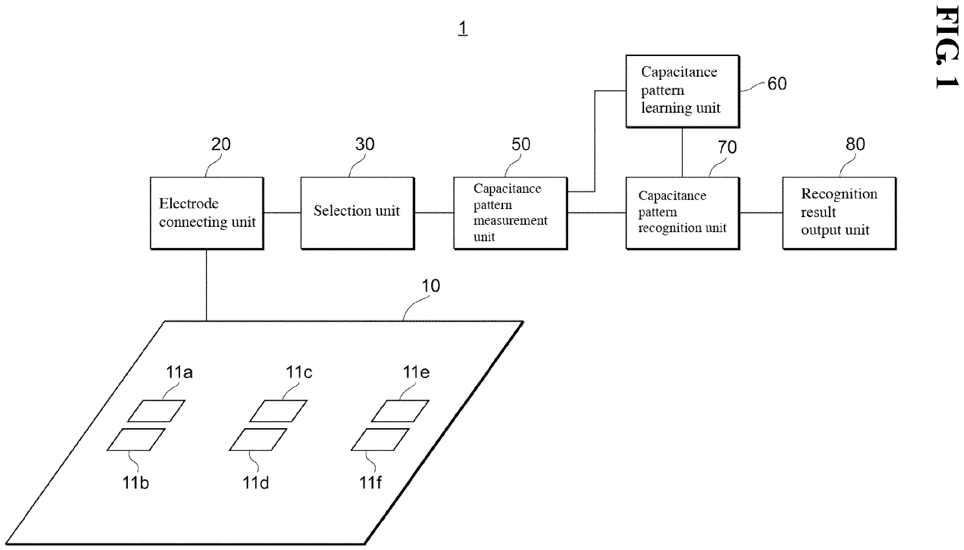

FIG. 1 is a diagram showing an overall concept of a state determination system 1 according to a first embodiment of the present invention. As shown in this figure, the state determination system 1 is provided with an electrode connecting unit 20, a selection unit 30, a capacitance pattern measurement unit 50, a capacitance pattern learning unit 60, a capacitance pattern recognition unit 70, and a recognition result output unit 80, and is connected to, via the electrode connecting unit 20, electrodes 11a, 11b, 11c, 11d, 11e, and 11f (hereinafter, simply referred to as "electrodes 11" if there is no need to distinguish the electrodes) of an electrode group 10. Note that the configuration of this system is not limited to the illustrated one, and a configuration is also possible in which the state determination system 1 includes the electrode group 10. Furthermore, two or more of the above-described units may also be physically or logically integrated into a single unit, and any of the units may also be physically or logically divided into two or more elements.

As the electrodes 11, conductors made of metal or the like that have a predetermined area may be used. If there are two electrodes (one electrode pair), the capacitance therebetween can be measured by connecting the electrode pair to a measurement circuit. As the electrodes 11, transparent members may also be used, and in this case, by providing the transparent electrodes 11 on a surface of a display device, a touch panel can be configured.

The selection unit 30 selects two of the plurality of electrodes 11 included in the electrode group 10. By sequentially selecting electrode pairs, the selection unit 30 may sequentially select a plurality of electrode pairs. For example, the selection unit 30 can sequentially select, as electrode pairs, three electrode pairs, namely, a pair of electrodes 11a and 11b, a pair of electrodes 11c and 11d, and a pair of electrodes 11e and 11f. The selection of electrode pairs by the selection unit 30 may be executed based on an electrode pair list that is stored in advance in a storage unit of the state determination system 1, may be executed in accordance with an input given by a user of the state determination system 1, or may be executed in random manner.

The capacitance pattern measurement unit 50 measures, based on the capacitances measured for the plurality of electrode pairs selected by the selection unit 30, a capacitance pattern for the plurality of electrode pairs selected by the selection unit 30. "Capacitance pattern" refers to a series of capacitance values measured for a plurality of electrode pairs. Specifically, as shown in FIG. 1, a case in which six electrodes 11a to 11f are provided as the electrode group 10 will be described. For example, when the selection unit 30 sequentially selects, from the six electrodes 11a to 11f, three electrode pairs, namely, the pair of electrodes 11a and 11b, the pair of electrodes 11c and 11d, and the pair of electrodes 11e and 11f, capacitances Cab, Ccd, and Cef for the respective electrode pairs selected by the selection unit 30 can be sequentially measured. Based on the series of capacitances (Cab, Ccd, and Cef) measured for the three electrode pairs, a capacitance pattern is measured.

Any method may be employed as the method for measuring capacitances. For example, by measuring a voltage when a predetermined electric charge is applied, capacitance measurement may also be possible. Alternatively, capacitance measurement may also be possible by configuring an LC oscillation circuit and measuring a frequency. The capacitance changes in accordance with the permittivity of an object between when the object is present in the vicinity of electrodes 11 and when it is not present in the vicinity of the electrodes 11. Accordingly, if an object is approaching one of the electrode pairs, the capacitance measured for this electrode pair changes in accordance with the permittivity of the object. Accordingly, by measuring a capacitance pattern for the plurality of electrode pairs, it is possible to determine whether or not an object is present in the vicinity of the plurality of electrodes 11, and detect, based on a determination result, an object's state (the presence of the object).

A capacitance pattern is regarded as data that is similar to an image; where an image shows the state of an object captured by using light, a capacitance pattern shows the state of an object captured by using capacitances. The capacitance pattern measurement unit 50 can measure the capacitances between electrode pairs selected by the selection unit 30. For example, if an AC voltage is applied to an electrode pair selected by the selection unit 30, the capacitance pattern measurement unit 50 can measure the capacitance by comparing the frequency of a voltage measured by the electrode pair with the frequency of the applied voltage.

If, for example, no object is present in the vicinity of the electrodes 11, a capacitance pattern measured by the capacitance pattern measurement unit 50 is affected by air present in the vicinity of the plurality of electrodes 11. In contrast, if an object is present, a capacitance pattern affected by the object is measured.

The capacitance pattern learning unit 60 performs machine learning using capacitance patterns measured by the capacitance pattern measurement unit 50 as learning data, and thereby obtains an ability to recognize capacitance patterns.

For example, the capacitance patterns measured for the same object located at a predetermined position from the plurality of electrodes 11 are substantially the same. At this time, if the object is moved slightly in any direction, the corresponding capacitance pattern will slightly change. Accordingly, using capacitance patterns generated based on data measured upon changing the positions of the object as learning data, it is possible to learn an ability to determine the position of an object, and detect the position of the object.

If measurement is performed for the same object located at a predetermined position from the plurality of electrodes 11 while changing its orientation, substantially the same pattern can be obtained for the same orientation. If the orientation of an object is slightly changed, the corresponding capacitance pattern will slightly change. Accordingly, by using, as learning data, capacitance patterns generated based on data measured while changing the orientations of objects, it is possible to learn an ability to determine the orientation of an object, and detect the orientation of the object.

If an object moves with respect to the plurality of electrodes 11, the measured pattern will change in accordance with the movement. By learning capacitance patterns before movement in combination with capacitance patterns after the movement, it is possible to obtain an ability to determine whether or not the object has moved, and detect the movement of the object.

After the completion of the learning by the capacitance pattern learning unit 60, a state is achieved in which a neural network constituting the capacitance pattern learning unit 60 has learned the capacitance patterns and has obtained the predetermined recognition ability. The capacitance pattern learning unit 60 duplicates or converts the trained neural network, for example, to generate information for reconfiguring (rebuilding) the trained neural network in such a manner that the capacitance pattern recognition unit 70 can use the obtained ability, and passes the generated information to the capacitance pattern recognition unit 70.

By recognizing a capacitance pattern using the ability obtained as a result of learning with the capacitance pattern learning unit 60, the capacitance pattern recognition unit 70 determines the state of an object that is present in the vicinity of the plurality of electrodes 11. The capacitance pattern recognition unit 70 receives the information for reconfiguring the trained neural network from the capacitance pattern learning unit 60, and reconfigures the trained neural network. Accordingly, the capacitance pattern recognition unit 70 can determine the object's state. In other words, a state of an object is determined by inputting a capacitance pattern measured by the capacitance pattern measurement unit 50 to the trained neural network and performing a calculation. For example, it is possible to determine a state such as a classification of an object's state, presence of an object, a position of the object, a type of the object, an orientation of the object, a material of the object, or a distribution of the object. Additionally, it is possible to obtain various abilities to identify an object's state that may cause a difference in a capacitance pattern, such as an ability to identify the above-described change, an ability to classify the change, and an ability to predict a change, for example.

The recognition result output unit 80 outputs, to the capacitance pattern recognition unit 70, a result of determination performed upon an input of a capacitance pattern. For example, when it is determined whether or not an object is present, if an identifier (ID) of the target to be identified can be determined, the ID may be output. Furthermore, if it is determined whether or not a specific target is present and information as to whether or not the specific target is present is to be detected, information indicating that "the target was detected" may be output. Furthermore, the identified state can also be visualized. For example, by displaying an image that shows the shape of an object, it is also possible for a viewer to recognize, by viewing the image, what this object is. By reflecting the orientation of the identified object on the image, it is also possible for the viewer to recognize, by viewing the image, the orientation of the object. By reflecting an identified movement on the image, the movement can also be visualized.

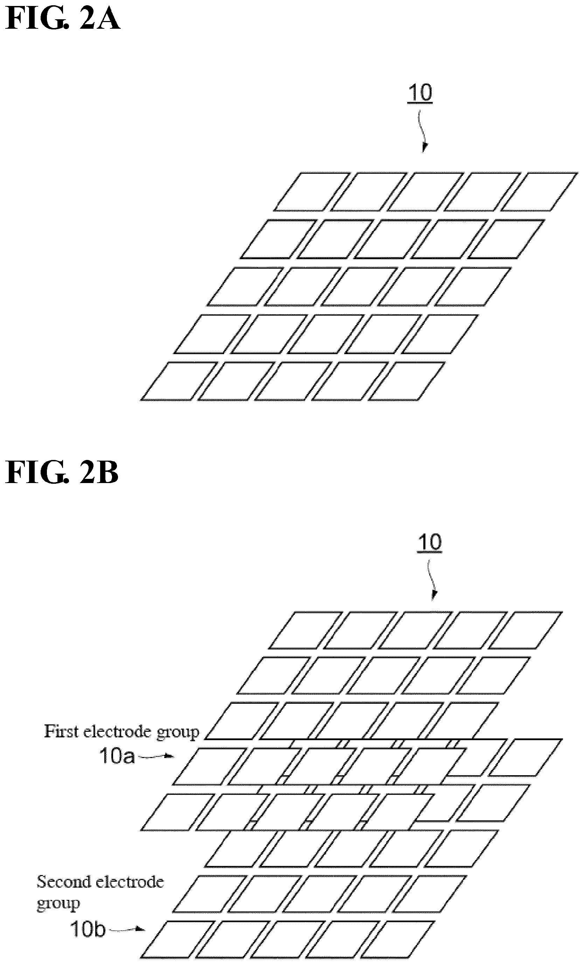

FIGS. 2A and 2B show examples of layouts of a plurality of electrodes 11. FIG. 2A shows a layout example in which the electrodes 11 are arranged side by side in a vertical-horizontal matrix. For example, if the electrodes 11 are arranged in an N×M matrix, the selection unit 30 may also select N×MC2 electrode pairs. At this time, the selection unit 30 may also include a switch matrix in which any one of the plurality of electrodes 11 arranged in the matrix can be selected and then switched to another one. If two electrodes 11 that are arranged close to each other, such as electrodes adjacent vertically or horizontally to each other, are combined with each other, the permittivity of an object that is present in the vicinity of these electrodes will exert a large influence. On the other hand, if two electrodes 11 that are apart from each other are selected, objects in a wide range will exert an influence.

Accordingly, if the capacitances of a plurality of electrode pairs are measured while changing an electrode to be combined with a specific electrode 11i, it is possible to obtain more detailed information regarding the presence of an object in a space in the vicinity of this specific electrode 11i. Furthermore, if another specific electrode 11j is used, it is possible to obtain more detailed information regarding the presence of an object in another space. By measuring the capacitances of a plurality of electrode pairs sequentially using specific electrodes such as other electrodes 11k, 11m, ..., it is possible to obtain more detailed information regarding the object's presence in a stepwise manner.

For example, there are three hundred types of combination of two electrodes selected from the electrodes arranged in 5×5 matrix shown in FIG. 2A. By measuring the capacitances using these combinations of electrodes, three hundred sets of capacitance data can be obtained. The capacitance pattern formed by the series of capacitance data reflects the state of an object that is present in the vicinity of the rectangular region in which the plurality of electrodes 11 are arranged. Note that, when forming a capacitance pattern, all (here, three hundred) or only some of the combinations of two electrodes selected from the plurality of electrodes 11 may be used.

FIG. 2B shows a layout example in which a first electrode group 10a and a second electrode group 10b face each other that are each constituted by a plurality of electrodes 11 arranged in matrixes. The selection unit 30 can select an electrode pair from the first electrode group, can select an electrode pair from the second electrode group, or can select a pair of electrodes from the respective first and second electrode groups.

In the layout shown in FIG. 2A, the state of an object that is present in a space relatively close to the electrode plane can be determined. In contrast, when, as shown in FIG. 2B, two groups of electrodes are arranged facing each other, the state of an object that is present anywhere in the space between the two electrode groups can be determined. For example, by selecting one electrode from each of the first and second groups and combining the selected electrodes together, it is easy to measure the influence of an object located at a position away from the electrode plane.

Note that the present invention is not limited to the examples shown in FIGS. 2A and 2B, and not all of the electrode pair combinations need to be used, instead any combination can be selected. Furthermore, the number of electrodes is given just as an example, and a suitable number of electrodes can be used. The electrodes 11 may have any shape and size, and may also be arranged at any positions. For example, a plurality of electrodes 11 may be arranged in a vehicle interior of an automobile while enclosing a passenger. As a result, the state of an object that is present in an enclosed space such as the vehicle interior can be determined, and the three-dimensional shape and the state are easily determined. Furthermore, the positions and the size of the electrodes 11 can be set based on the shape of a space to be subjected to determination and the state of an object. As in a later-described example of a robot hand, a movable means may also be used to change the positions and orientations of the electrodes 11.

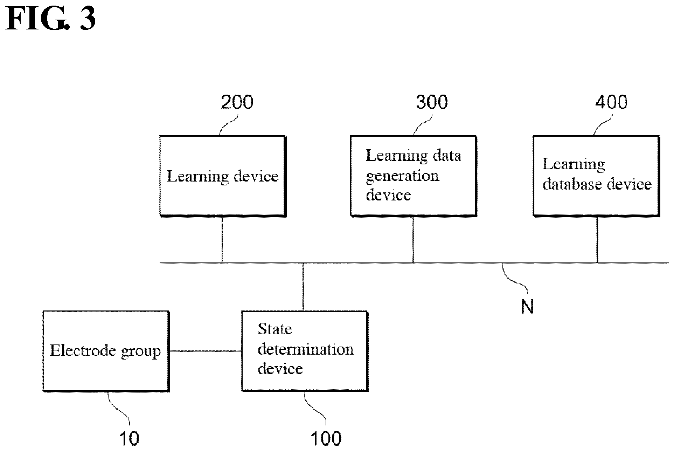

FIG. 3 is a diagram illustrating an example of a specific system configuration of the state determination system 1 according to the first embodiment. As shown in this figure, in the state determination system 1, a state determination device 100 connected to the electrode group 10, a learning device 200, a learning data generation device 300, and a learning database device 400 are connectable to each other via a network N.

The state determination device 100 substantially includes the electrode connecting unit 20, the selection unit 30, the capacitance pattern measurement unit 50, the capacitance pattern recognition unit 70, and the recognition result output unit 80 that are shown in FIG. 1, and is configured to determine, based on a capacitance pattern of the plurality of electrodes 11 included in the electrode group 10, a state of an object that is present in the vicinity of the plurality of electrodes 11.

The learning device 200 substantially includes the capacitance pattern learning unit 60 shown in FIG. 1. The learning device 200 performs machine learning using learning data that contains capacitance patterns stored in the learning database device 400 to train a neural network, and obtains an ability to recognize the state of an object that is present in the vicinity of the plurality of electrodes based on the capacitance patterns. The ability obtained by the learning device 200 is passed to the state determination device 100 directly or via the learning database device 400.

The learning data generation device 300 generates learning data or teaching data that is required depending on the type of an ability to be obtained, and stores the generated data in the learning database device 400. In the system configuration shown in FIG. 3, when generating learning data, the learning data generation device 300 acquires, via the state determination device 100, a capacitance pattern of the plurality of electrodes 11 included in the electrode group 10 and state of the object that is present in the vicinity of the electrodes 11 when this capacitance pattern is obtained, and generates, for example, learning data based on the capacitance pattern and teaching data based on the object's state at this time.

If, for example, an ability to classify an object's state is to be obtained, capacitance patterns can be measured for a plurality of states of an object and can be set as learning data. In other words, data to serve as learning data that is obtained by measuring and storing capacitance patterns for a plurality of states of an object is used as learning data. As for teaching data, for each capacitance pattern, information for identifying an object's state is stored as teaching data in association with the learning data. At this time, the teaching data may also be associated with data other than the learning data, or the teaching data may also be contained in the learning data. If objects only need to be classified into a plurality of groups, teaching data does not need to be included.

If an ability to classify a change in an object's state is to be obtained, time series data of a capacitance pattern is needed, and thus, for example, learning data containing teaching data that indicates content of the change is generated. If an ability to predict an object's state is to be obtained, learning to predict an object's state is performed using, for example, time series data of a capacitance pattern that contains teaching data regarding the content of the object's state.

Learning data can be automatically generated using a learning data generation program installed in the learning data generation device 300. Because, for example, deep learning requires a large amount of data, automation of a series of operations from acquisition of a capacitance pattern to its registration to the learning database device 400 can largely reduce the number of operations. By, for example, bringing an object into the vicinity of the plurality of electrodes 11, instructing a robot to change the position or orientation of the object, and obtaining capacitance patterns for various positions, orientations, and directions in association with the position, orientation, and direction of the object at this time, it is possible to automatically generate learning data and teaching data without performing human operations. The learning data generation device 300 may also be subjected to manual operations to generate learning data. Furthermore, any type of information that may relate to learning may also be combined with each other to serve as learning data or teaching data.

Note that, since a capacitance pattern is measured according to the positional relationship between the electrodes 11 and an object, the layout of the electrodes 11 may affect the leaning result. Accordingly, learning data may also contain information that relates to the layout of the electrodes. Furthermore, a configuration is also possible in which the state determination device 100 or the learning device 200 has the functions of the learning data generation device 300. In this case, by storing data output from the state determination device 100 or the learning device 200 in the learning database device 400, inputting the data to the learning device 200, or using the data, it is possible to use the data as learning data or teaching data.

The learning database device 400 has a function to store learning-related data and a learning-related program, such as learning data or a learning program generated by the learning data generation device 300, and setting thereof, for example. The learning data or teaching data generated by the learning data generation device 300 is passed to the learning database device 400 and is stored as learning data-related information.

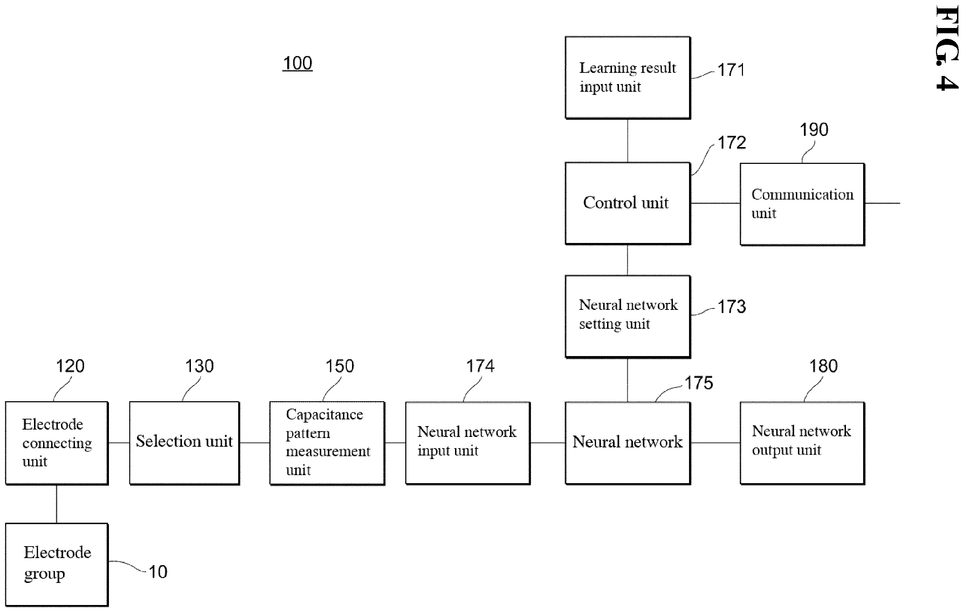

FIG. 4 is a block diagram showing a configuration of the state determination device 100 according to the present embodiment. Of the constituent components shown in this figure, an electrode connecting unit 120, a selection unit 130, a capacitance pattern measurement unit 150, and a neural network output unit 180 respectively have functions that correspond to the electrode connecting unit 20, the selection unit 30, the capacitance pattern measurement unit 50, and the recognition result output unit 80 that are shown in FIG. 1, and thus descriptions thereof are omitted. A learning result input unit 171, a control unit 172, a neural network setting unit 173, a neural network input unit 174, and a neural network 175 have functions that corresponds to the capacitance pattern recognition unit 70 shown in FIG. 1.

The learning result input unit 171 receives information relating to an ability obtained as a result of learning of the learning device 200, and inputs the received information to the state determination device 100. Here, data is input that is required to reconfigure the trained neural network output from the learning device 200. The control unit 172 controls processing in each of the constituent components of the state determination device 100, and is configured, for example, to output a capacitance pattern acquired from the electrode group 10 to the learning data generation device 300 via a communication unit 190 in accordance with an instruction from the learning data generation device 300. Furthermore, the control unit 172 passes, to the neural network setting unit 173, the data required to reconfigure the trained neural network input from the learning result input unit 171. The neural network setting unit 173 reconfigures the neural network 175 based on this data. Accordingly, the neural network 175 can reproduce the ability obtained as a result of learning of the learning device 200.

The neural network input unit 174 acquires a capacitance pattern from the capacitance pattern measurement unit 15, and inputs the acquired capacitance pattern to the neural network 175. The neural network 175 performs calculation on the input capacitance pattern, and outputs a calculation result. This calculation result corresponds to a determination result of the state of an object that is present in the vicinity of the plurality of electrodes 11, that is, an object recognition result, and this recognition result is output from the neural network output unit 180. Note that, in place of the neural network, a suitable learning module that can perform machine learning may also be used. As a result of calculation of the neural network 175, the type of an object may also be determined, and an ID assigned to the type of the object may also be output.

The neural network output unit 180 can output, as an object recognition result, a state such as, for example, a classification of a state of an object that is present in the vicinity of the plurality of electrodes 11, presence of an object, a position of the object, a type of the object (object type), an orientation of the object, a material of the object, or a distribution of the object. The recognition result to be output is not limited to them, and additionally a change in an object's state, a classification of the change, a prediction of the change, or the like can be output, for example. Note that "object type" refers to individual types when, for example, there are a plurality of types of objects (for example, an apple and a pear) that have similar shapes, weights, and sizes. Furthermore, the neural network output unit 180 may also generate an image or a video based on the determined state of an object, and show a user the visualized state of the object.

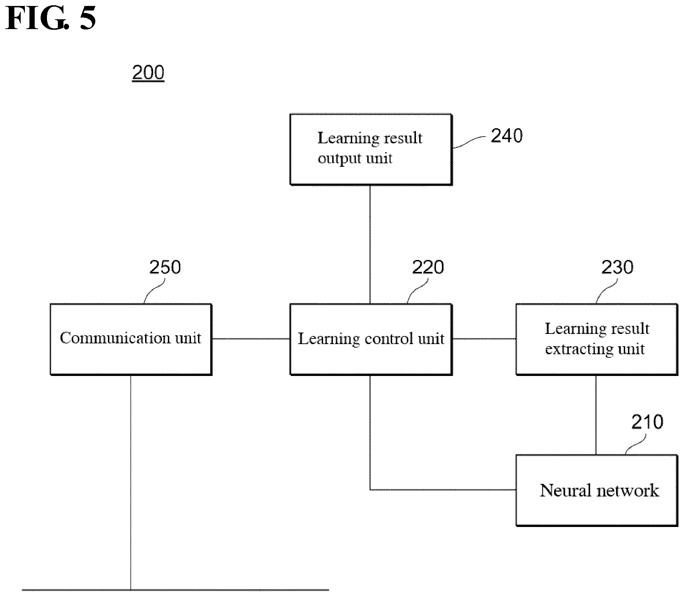

FIG. 5 is a block diagram showing a configuration of the learning device 200 according to the present embodiment. As shown in this figure, the learning device 200 includes a neural network 210, a learning control unit 220, a learning result extracting unit 230, a learning result output unit 240, and a communication unit 250, which are connectable to each other via a network (including an internal bus or the like).

The neural network 210 has a function as a neural network, and can obtain a predetermined ability through learning. The learning device 200 trains the neural network 210 using learning data or teaching data stored in the learning database device 400 so that the neural network 210 obtains a predetermined ability. The neural network 210 may be a neural network in which a capacitance pattern vector is input to an input layer, and a vector indicating an object's state is output from an output layer. Note that, in place of the neural network, a learning module that can perform machine learning may also be used.

The learning control unit 220 executes a learning program to control machine learning of the learning device 200. By executing the learning program, the learning control unit 220 executes learning in accordance with a predetermined learning request received from a user, and controls the neural network 210 to learn and obtain a predetermined ability using learning data. For example, by learning using a learning program with deep learning technique, the neural network 210 can obtain a predetermined ability. For learning of a static state of an object, a convolutional neural network (CNN), which may be used for image recognition, may also be used as the neural network 210. Also, for learning of a dynamic state of an object, a recurrent neural network (RNN) may also be used.

The learning result extracting unit 230 extracts a result of learning of the neural network 210. The learning result output unit 240 outputs the learning result to the outside via the communication unit 250. In the present embodiment, as a result of learning, the learning device 200 can obtain, for example, an ability to determine an object's state.

The learning device 200 may be implemented by executing a predetermined program on a general-purpose computer. At this time, a computer provided with a high-speed CPU is preferably used because of a large amount of throughput of learning processing. If a GPU is used, repeat operation processing can be accelerated. Using a server device on which a blade computer is rack-mounted allows parallelization of the processing to increase the throughput. If requests are received from a plurality of clients, the server device can be multiplexed to increase the degree of parallelization.

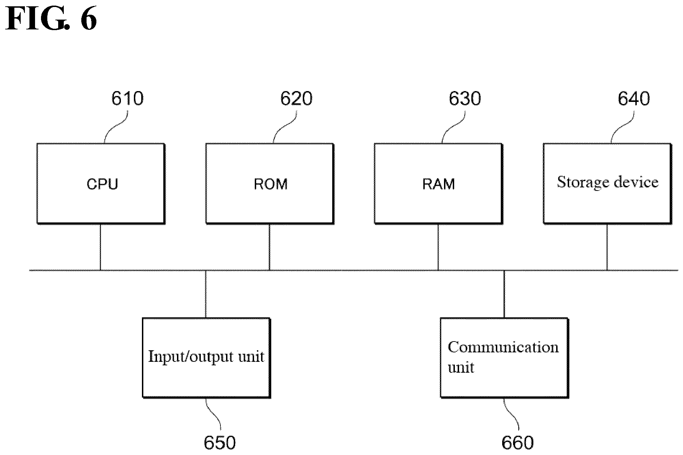

FIG. 6 is a block diagram showing an example of a hardware configuration of the constituent components of the state determination system 1. As shown in this figure, a general-purpose or a dedicated purpose computer provided with a CPU 610, memories such as a ROM 620 and a RAM 630, a storage device 640 for storing various types of information, an input/output unit 650, a communication unit 660, and a network or a bus for connecting them can be applied to the devices such as the state determination device 100, the learning device 200, the learning data generation device 300, and the learning database device 400. The devices can realize the above-described functional blocks by, for example, the CPU 610 executing a predetermined program stored in a memory or the storage device 640. The program can be installed or loaded into the computer that configures the devices as a result of being downloaded through various types of storage media such as an optical disk, for example, a CD-ROM, a magnetic disk, or a semiconductor memory, or a communication network or the like.

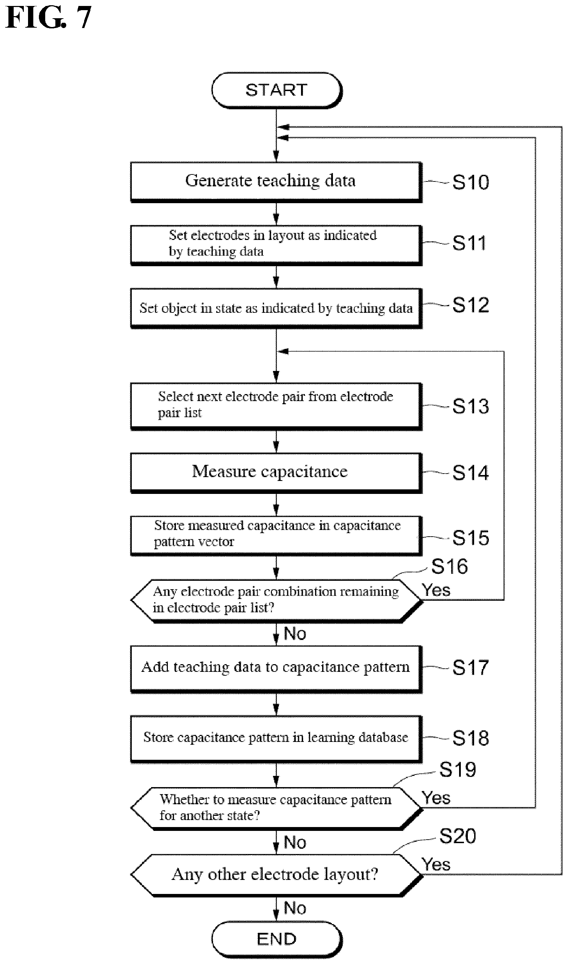

The following will describe a processing flow of the system according to the present embodiment with reference to FIGS. 7 to 9.

FIG. 7 is a flowchart of learning data generation processing executed by the learning data generation device 300 according to the present embodiment. The learning data generation processing is executed prior to execution of object recognition processing by the state determination device 100. First, the learning data generation device 300 generates teaching data that contains information relating to the state of an object that is present in the vicinity of the plurality of electrodes 11 (step S10). The teaching data may also contain information relating to a layout of the plurality of electrodes 11. Here, the information relating to an object's state may also be input by a user to an input unit (not-shown) of the learning data generation device 300. The user inputs, as the information relating to an object's state, numerical values that indicates the position, angle, type, material, and distribution of an object, for example. The learning data generation device 300 may also form vectors that indicate the information relating to an object's state based on the input numerical values, and add the formed vectors to the teaching data. Furthermore, the information relating to a layout of the electrodes 11 may also be input by a user to the input unit of the learning data generation device 300. The user inputs, for example, the size, positions and angles, and the material of the electrodes, and the learning data generation device 300 may also form vectors that indicate the information relating to a layout of the electrodes based on the numerical values, and add the formed vectors to the teaching data. Furthermore, if information relating to a layout of the electrodes is given to the electrode group 10, this information may also be referenced.

The teaching data may also contain an image or a video as the information relating to an object's state. By showing an object's state in an image or a video, it is possible to objectively show an object's state without the need of a user converting the object's state into numerical values, execute highly objective neural network learning, and obtain a more versatile learning result of the neural network.

If the plurality of electrode 11 are provided so as to be movable or rotatable, a user, a robot, or the state determination device 100 sets, in accordance with an instruction of the learning data generation device 300, the electrodes 11 in a layout as indicated by the teaching data (step S11). By more flexibly setting a layout of the electrodes 11 with respect to an object without using a fixed layout of the electrodes 11, it is possible to generate learning data on which an object's state is reflected more specifically. Note that, if the plurality of electrodes 11 are fixed, the step of setting the electrodes 11 in a layout as indicated by the teaching data can be omitted.

The learning data generation device 300 sets an object's state to the state indicated by information relating to the object's state that is contained in the teaching data (step S12). Then, the learning data generation device 300 selects an electrode pair from the electrode group 10 with reference to an electrode pair list (step S13). The learning data generation device 300 applies, for example, a predetermined voltage to the selected electrode pair to measure a capacitance (step S14), and stores the measured capacitance in a capacitance pattern vector (step S15). The capacitance pattern vector is a vector that contains the measured capacitance value as an element.

The learning data generation device 300 determines whether or not, of the electrode pairs included in the electrode pair list, any electrode pair combination that has not been selected remains (step S16). If any electrode pair combination that has not been selected remains (Yes in step S16), the procedure returns to step S13, where a new electrode pair is selected, a capacitance for the new electrode pair is measured, and the measured capacitance is added to the capacitance pattern vector. If no electrode pair combination that has not been selected remains (No in step S16), the teaching data is added to the obtained capacitance pattern (step S17). Then, the learning data generation device 300 stores the obtained capacitance pattern in association with the teaching data in the learning database device 400 (step S18).

The learning data generation device 300 determines whether or not a capacitance pattern for another state of the object is to be measured (step S19). If a capacitance pattern for another state of the object is to be measured (Yes in step S19), the learning data generation device 300 returns to the processing in step S10, where the above-described processing is executed again on another object. On the other hand, if no capacitance pattern for another state of the object is to be measured (No in step S19), the learning data generation device 300 determines whether or not there is another electrode layout of the electrode group 10 (step S20). If there is another electrode layout of the electrode group 10 (Yes in step S20), the learning data generation device 300 executes again the above-described processing on the other electrode layout. On the other hand, if there is no other electrode layout of the electrode group 10 (No in step S20), the learning data generation processing of the learning data generation device 300 is ended.

According to the learning data generation device 300 of the present embodiment, it is possible to generate learning data containing a capacitance pattern for an object that was measured with respect to the electrode group 10, and teaching data containing information relating to an object's state in association with each other. As a result of performing learning using the learning data containing a capacitance pattern and the teaching data, the learning device 200 can obtain an ability to determine a more detailed state of the object than in a case where a change in the capacitance between a single pair of electrodes is used.

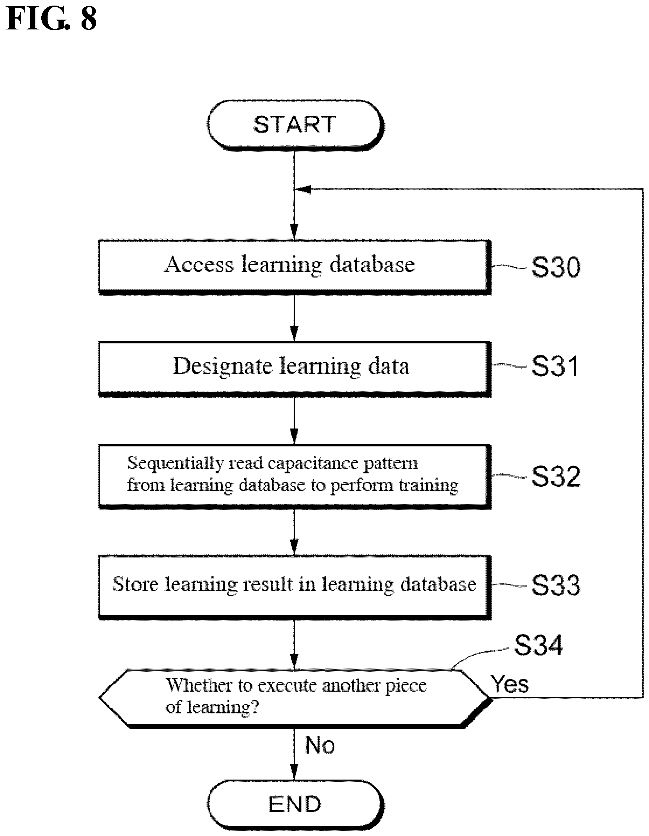

FIG. 8 is a flowchart of learning processing performed by the learning device 200 according to the present embodiment. Here, description will be given assuming that the learning processing is executed after the execution of the learning data generation processing by the learning data generation device 300, but the present invention is not limited to this, and learning processing may also be executed, for example, in parallel to the learning data generation processing executed by the learning data generation device 300. First, the learning device 200 accesses the learning database device 400 (step S30), and designates learning data to be used in learning (step S31). The learning device 200 sequentially reads capacitance patterns contained in the designated learning data, and trains the neural network 210 (step S32). The learning of the neural network 210 may be, for example, learning executed using a backpropagation method. In the learning device 200, the learning result extracting unit 230 extracts a result of the learning of the neural network 210, and the learning result output unit 240 outputs the learning result to the learning database device 400 or a suitable external storage device via the communication unit 250 (step S33).

Ultimately, the learning device 200 determines whether or not another piece of learning is to be executed (step S34). If another piece of learning is to be executed (Yes in step S34), a different piece of learning data is designated and the neural network is again trained. If no other piece of learning is to be executed (No in step S34), the learning processing is ended.

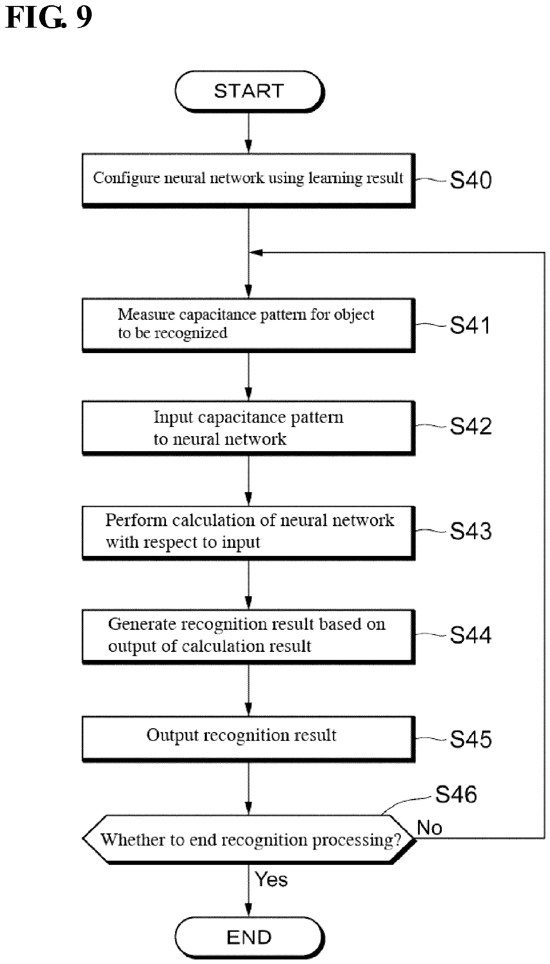

FIG. 9 is a flowchart of object recognition processing executed by the state determination device 100 according to the present embodiment. The object recognition processing is processing that is typically executed after the execution of training of the neural network by the learning device 200. First, the state determination device 100 accesses the learning database device 400, inputs the learning result output by the learning device 200 using the learning result input unit 171, and configures the neural network 175 using the neural network setting unit 173 (step S40). Accordingly, the neural network 175 can reproduce the ability obtained as a result of the learning of the learning device 200.

If there is an object to be recognized in the vicinity of the plurality of electrodes 11, the state determination device 100 selects a plurality of electrode pairs from the plurality of electrodes 11, and measures a capacitance pattern for the object (step S41), and the neural network input unit 174 inputs the capacitance pattern to the neural network 175 (step S42). The neural network 175 performs calculation with respect to this input (step S43), and the neural network output unit 180 outputs a calculation result. The state determination device 100 determines an object's state based on the output calculation result, that is, generates an object recognition result (step S44), and outputs the object recognition result (step S45). The recognition result to be output is, for example, presence of an object, the presence of an object, a position of the object, a type of the object, an orientation of the object, a material of the object, a distribution of the object, or the like, but is not limited to them.

The state determination device 100 determines whether or not the object recognition processing is to be ended (step S46), and if the object recognition operation is not to be ended (No in step S46), the capacitance pattern is updated, and the object recognition processing by the neural network 175 is executed again. If the object recognition operation is to be ended (Yes in step S46), the object recognition processing is ended.

As described in detail above, according to the state determination device 100 of the present embodiment, by selecting a plurality of electrode pairs from the plurality of electrodes 11 included in the electrode group 10 to measure a capacitance pattern, and determining an object's state based on the capacitance pattern, it is possible to determine the object's state in more detail than in a case where a change in the capacitance between a single pair of electrodes is used.

### Second Embodiment

Hereinafter, an embodiment in which the state determination system 1 is applied to a robot hand will be described as a second embodiment.

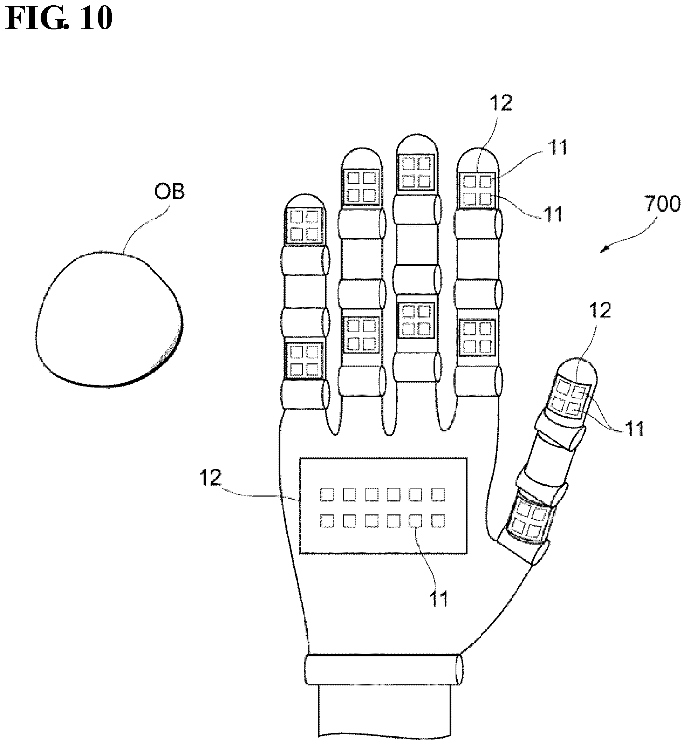

FIG. 10 is a diagram showing a situation in which the electrodes 11 are provided on a robot hand 700. As shown in this figure, according to the present embodiment, a plurality of electrodes 11 and a plurality of guard electrodes 12 are provided on the robot hand 700. As descried later, the state determination device 100 measures a capacitance pattern for the plurality of electrodes 11 provided on the robot hand 700, and determines the state of an object OB that is to be held or operated by the robot hand 700. Here, the robot hand 700 is typically constituted by a multi-jointed arm section and a multi-fingered and multi-jointed hand section provided at the leading end of the arm section. Note that a mode in which the plurality of electrodes are provided on the robot hand 700 is taken as an example, and the plurality of electrodes may be provided at any position without being limited to the robot hand 700.

The electrodes 11 are made of planar conductors, and are provided on the palm side of the robot hand 700. In the present example, the electrodes 11 are provided at the ends of the finders, in spaces between the second and third joints of the respective fingers, and on the palm, while being arranged side by side in a matrix. The guard electrodes 12 are made of planar conductors, and are arranged enclosing at least some of the plurality of electrodes 11. The guard electrodes 12 are provided on the palm side of the robot hand 700, and are arranged, in the present example, at the ends of the finders, in spaces between the second and third joints of the respective fingers, and on the palm, while enclosing the electrodes 11. Note that layouts of the electrodes 11 and the guard electrodes 12 shown in this figure are taken as examples, and suitable layouts of the electrodes 11 and the guard electrodes 12 may be employed. The state of the object OB in a smaller area can be determined the smaller the electrodes 11 are, and the state of the object OB in a larger area can be determined the larger the electrodes 11 are.

The object OB is arranged in front of the robot hand 700. The object OB is a suitable object, and may also be liquid or air. The object OB may also be a living object such as a human body, or a non-living object such as industrial tool. Furthermore, the object OB may also be any of a conductive material, a semiconductor material, and an insulating material, or may also be a transparent object or a non-transparent object.

By providing the plurality of electrodes 11 on the robot hand 700 and determining the state of an object OB based on a capacitance pattern of the plurality of electrodes 11, the state of the object to be held or operated by the robot hand 700 can be determined, and more precise operation of the robot hand 700 can be assisted. For example, when holding an object using the robot hand 700, the robot hand 700 can be operated to uniformly bring a plurality of fingers thereof close to the object.

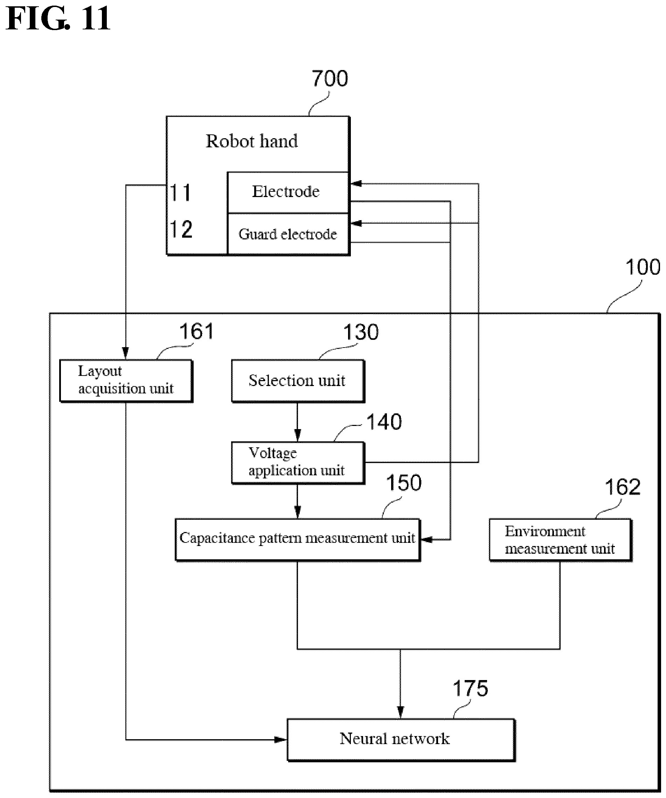

FIG. 11 is a functional block diagram showing the state determination device 100 according to the second embodiment. The state determination device 100 is connected to the electrodes 11 and the guard electrodes 12, and is provided with the selection unit 130, the voltage application unit 140, the capacitance pattern measurement unit 150, the neural network 175, a layout acquisition unit 161 and an environment measurement unit 162. Note that the state determination device 100 is provided with, as a physical configurations, an input unit (such as a keyboard or a mouse), an output unit (such as a liquid display device), a calculation unit (such as a CPU or a GPU) that corresponds to a hardware processor, and a storage unit (such as an HDD, an SSD, or a semiconductor memory) that corresponds to a memory. The functional blocks shown in this figure indicate the functions that are exerted using the physical configurations of the state determination device 100, and do not necessarily correspond to the physical configurations in one-to one correspondence.

According to the present embodiment, the electrodes 11 and the guard electrodes 12 are provided on the robot hand 700, and all the electrodes 11 and the guard electrodes 12 are provided so as to be movable and rotatable. The selection unit 130 and the capacitance pattern measurement unit 150 have the same functions as those described with reference to FIG. 4.

The voltage application unit 140 applies a predetermined voltage to a plurality of electrode pairs selected by the selection unit 130. The voltage application unit 140 applies a reference voltage to one of the electrodes of each of the electrode pairs selected by the selection unit 130 and to the guard electrodes 12. Here, the reference voltage may be a ground potential but may also be, without being limited to, a suitable potential. The voltage application unit 140 may also apply a DC voltage or an AC voltage to the electrode pairs selected by the selection unit 130.

By the voltage application unit 140 applying a reference voltage to one of the electrodes of each of the electrode pairs and the guard electrodes 12, the electric field is shielded by the guard electrodes 12, and a capacitance pattern for only one of the two sides of the electrodes 11 can be measured, making it possible to restrict the range for which the state of the object OB is to be determined. Accordingly, it is possible to determine the state of the object OB arranged on the palm side of the robot hand 700, without being affected by, for example, the robot hand 700 itself or an object arranged on the back side of the robot hand 700. Note that a configuration is also possible in which, by applying a reference voltage to some of the electrodes 11, some of the electrodes 11 function as the guard electrodes 12.

The layout acquisition unit 161 acquires a layout of the plurality of electrodes 11. Since the plurality of electrodes 11 according to the present embodiment are provided on the robot hand 700, the layout acquisition unit 161 may acquire the angles of the joints of the robot hand 700, and may calculate a layout of the plurality of electrodes 11 on the robot hand 700. The neural network 175 is a neural network that is trained to determine the state of an object that is present in the vicinity of the robot hand 700, using a capacitance pattern measured by the capacitance pattern measurement unit 150 and a layout of the plurality of electrodes 11 acquired by the layout acquisition unit 161 as input data.

The environment measurement unit 162 measures environmental data relating to an environment when the capacitance pattern measurement unit 150 measures a capacitance pattern of the plurality of electrodes 11. Here, "environmental data" refers to a physical amount that characterizes the environment in which the plurality of electrodes 11 are arranged. "Environmental data" specifically includes data relating to an environment that may causes a change in capacitance. In the present embodiment, "environmental data" includes data on at least one of humidity and electromagnetic noise. A change in humidity and the intensity of electromagnetic noise brings a change in permittivity or a change in the electric state of electric circuits that constitute the capacitance pattern measurement unit 150, and may cause a change in the capacitance to be measured. The neural network 175 may determine the state of an object, using the capacitance pattern measured by the capacitance pattern measurement unit 150 and the environmental data measured by the environment measurement unit 162 as input data. The determination result is output by the neural network output unit 180 (not-shown in FIG. 11).

The environment measurement unit 162 may also continuously measure environmental data while the state determination device 100 executes state determination processing. By measuring environmental data, it is possible to perform state determination with consideration given to the environment dependence of a capacitance pattern, thus making it possible to reduce an error in state determination that may be caused by a change in a measurement environment.

In the present embodiment, the environment measurement unit 162 measures, as environmental data, humidity and electromagnetic noise. Accordingly, it is possible to perform state determination with consideration given to the dependence on humidity or electromagnetic noise that may especially affect a capacitance pattern, and reduce an error in the state determination that may be caused by a change in humidity and the intensity of electromagnetic noise.

As the neural network 175 of the state determination device 100, a trained neural network is used that is trained by the learning device 200 so as to have an ability to determine the object's state. As the learning method, similar to the first embodiment, learning can be executed using learning data that was generated by the learning data generation device 300 and stored in the learning database device 400.

As a specific example, in a state in which the robot hand 700 is set in a predetermined layout with respect to an object OB prepared in a predetermined state with respect to the robot hand 700, and the plurality of electrodes 11 and the plurality of guard electrodes 12 are set in predetermined positions, the learning data generation device 300 forms a capacitance pattern measured for a plurality of selected electrode pairs to generate learning data, and stores the generated learning data in association with teaching data in the learning database device 400. The layout of the electrodes may be set as indicated by an electrode layout list stored in advance in the storage unit of the learning data generation device 300, may be set in accordance with an input by a user of the learning data generation device 300, or may be set in a random manner. The learning data generation device 300 may also add the layout of the plurality of electrodes 11 acquired by the layout acquisition unit 161 or the environmental data measured by the environment measurement unit 162 to the learning data or the teaching data.

The learning device 200 can perform machine learning using learning data and teaching data stored in the learning data generation device 300 to obtain a trained neural network having a predetermined ability. For example, even if a measurement environment of a capacitance pattern changes, it is possible to perform state determination with consideration given to the environment dependence of the capacitance pattern by performing machine learning using learning data that contains environmental data, and obtain a learning result with which an error in the state determination can be reduced.

### Third Embodiment

Hereinafter, an embodiment in which the state determination system 1 is applied to a seat of an automobile or the like will be described as a third embodiment.

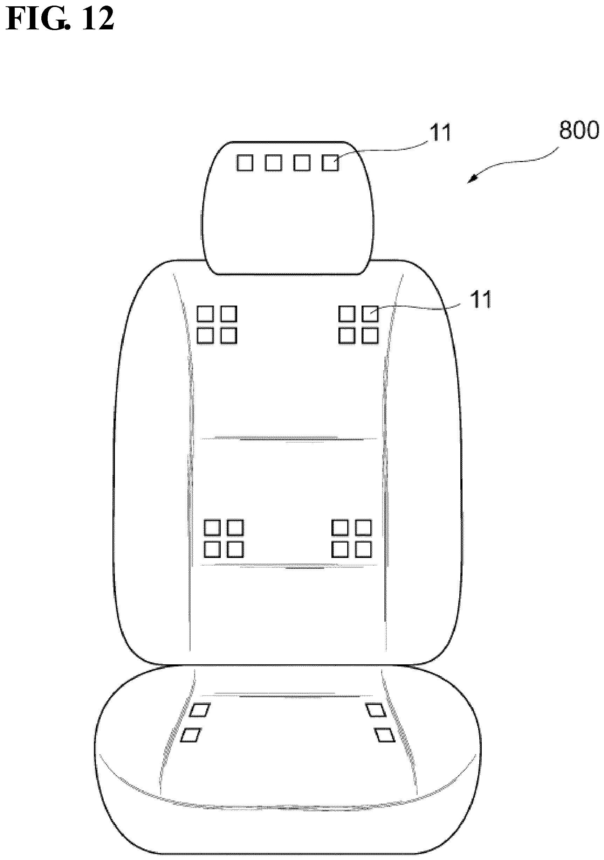

FIG. 12 is a diagram showing a situation in which the plurality of electrodes 11 are provided on a seat 800, according to the third embodiment. The state determination system of the third embodiment differs from that of the second embodiment in that the plurality of electrodes 11 are provided on the seat 800, and no guard electrode 12 are provided. Other configurations of the state determination system according to the third embodiment have the same as the configurations of the state determination system according to the second embodiment.

By providing the plurality of electrodes 11 on the seat 800, measuring a capacitance pattern, and determining an object's state using a trained neural network, it is possible to determine details of the state of a person seated on the seat 800, such as physical size and orientation. For example, by determining the condition of an automobile driver seated on a seat of an automobile, it is possible to determine whether or not the driver is awake (whether or not the driver is falling asleep). If it is determined that the driver is not awake, a warning tone or the like is generated for example, so that an accident can be avoided.

The present invention may also be executed in a configuration different from the above-described first, second, and third embodiments. For example, the plurality of electrodes 11 may also be mounted on a machine tool or the like to serve as, for example, a contact preventing sensor for determining an approach of a person or an object. Furthermore, the plurality of electrodes 11 may also be arranged on the windscreen of an automobile to serve as a raindrop sensor for determining the state of rain drops in rainfall. In this case, the plurality of electrodes 11 are preferably made of transparent electrodes. Furthermore, for example, the plurality of electrodes 11 may also be arranged on the front panel or bottom plate of an automobile to serve as a road surface sensor for determining the state of a road surface. Furthermore, for example, the plurality of electrodes 11 may also be arranged on a door handle of an automobile, and may determine a hand approaching the door and determine whether or not it is an action of the hand that is intended to lock or unlock the door, thereby allowing locking or unlocking of the door. Furthermore, for example, the plurality of electrodes 11 may also be arranged in the vicinity of the passenger doorway of a bus or near the luggage carrier outlet of a truck, and may determine the state of passengers or luggage. Furthermore, for example, the plurality of electrodes 11 may also be arranged inside an automobile, and may determine a gesture of a hand to give an instruction to start operation of an audio device, open/close a door or a trunk, open/close power windows, or the like. Furthermore, for example, the plurality of electrodes 11 may also be arranged outside an automobile to serve as an anticrime sensor for determining a tool used in an illegal behavior such as vehicle theft, or an operation involved by the illegal behavior.

The above-described embodiments are provided for ease of understanding of the present invention, rather than interpreting the present invention in a restrictive manner. The constituent elements of the embodiments and the layout, the material, the condition, the shapes and the sizes thereof are not limited to the exemplified ones, and may be modified as appropriate. Furthermore, configurations shown in different embodiments may also be partially replaced or combined with each other.

Furthermore, some or all of the above-described embodiments may also be described in "Supplementary Notes" below, but the present invention is not limited to them.

### Supplementary Note 1

A state determination device comprising:
at least one memory; and at least one hardware processor connected to the memory,
wherein the hardware processor is configured to:
   select a plurality of electrode pairs from a plurality of electrodes;
   measure a capacitance pattern for the selected plurality of electrode pairs; and
   determine a state of an object based on the measured capacitance pattern using a trained neural network.

### Supplementary Note 2

A state determination method for determining a state of an object that is present in the vicinity of a plurality of electrodes, comprising the steps of:
selecting, by at least one hardware processor, a plurality of electrode pairs from a plurality of electrodes;
measuring, by the hardware processor, a capacitance pattern for the selected plurality of electrode pairs; and
determining, by the hardware processor, a state of an object based on the measured capacitance pattern using a trained neural network.

## Claims

1. A state determination device for determining a state of an object that is present in the vicinity of a plurality of electrodes, comprising:
a selection unit configured to select a plurality of electrode pairs from the plurality of electrodes;
a capacitance pattern measurement unit configured to measure a capacitance pattern for the plurality of electrode pairs selected by the selection unit; and
a neural network that is trained to determine a state of the object based on the capacitance pattern.

2. The state determination device according to claim 1, further comprising:
a layout acquisition unit configured to acquire a layout of the plurality of electrodes,
wherein the neural network determines a state of the object based on data that further contains the layout of the plurality of electrodes acquired by the layout acquisition unit.

3. The state determination device according to claim 1 or 2, further comprising:
a voltage application unit configured to apply a predetermined voltage to each of the plurality of electrode pairs selected from the plurality of electrodes by the selection unit; and
a guard electrode arranged enclosing at least some of the plurality of electrodes,
wherein the voltage application unit applies a reference voltage to one of the electrodes of each of the electrode pairs selected from the plurality of electrodes by the selection unit, and to the guard electrode.

4. The state determination device according to any one of claims 1 to 3, further comprising:
an environment measurement unit configured to measure environmental data that relates to an environment when a capacitance pattern for the plurality of electrode pairs is measured by the capacitance pattern measurement unit,
wherein the neural network determines a state of the object based on data that further contains the environmental data measured by the environment measurement unit.

5. The state determination device according to claim 4,
wherein the environmental data measured by the environment measurement unit contains data on at least one of humidity and electromagnetic noise.

6. The state determination device according to any one of claims 1 to 5,
wherein the neural network determines at least one of presence of the object, a position of the object, an angle of the object, a type of the object, a material of the object, and a distribution of the object.

7. The state determination device according to any one of claims 1 to 6,
wherein the plurality of electrodes are provided on a robot hand.

8. The state determination device according to any one of claims 1 to 6,
wherein the plurality of electrodes are provided on a seat.

9. A learning device for training a neural network that is used in the state determination device according to any one of claims 1 to 8, the learning device comprising,
a learning control unit configured to perform control such that the neural network is trained using learning data that contains a capacitance pattern for a plurality of electrode pairs selected from the plurality of electrodes.

10. The learning device according to claim 9,
wherein the learning data contains data that relates to a layout of the plurality of electrodes.

11. The learning device according to claim 9 or 10,
wherein the learning data contains environmental data that relates to an environment when the capacitance pattern is measured.

12. The learning device according to any one of claims 9 to 11,
wherein the learning control unit trains the neural network using the learning data and teaching data, and
the teaching data contains an image or a video that shows a state of the object.

13. A state determination method for determining a state of an object that is present in the vicinity of a plurality of electrodes, the method comprising:
a first step of selecting a plurality of electrode pairs from the plurality of electrodes;
a second step of measuring a capacitance pattern for the plurality of electrode pairs selected in the first step; and
a third step of determining, with a trained neural network, a state of the object based on the capacitance pattern measured in the second step.

14. A program for causing a computer provided in a state determination device for determining a state of an object that is present in the vicinity of a plurality of electrodes to function as:
a selection unit configured to select a plurality of electrode pairs from the plurality of electrodes;
a capacitance pattern measurement unit configured to measure a capacitance pattern for the plurality of electrode pairs selected by the selection unit; and
a neural network that is trained to determine a state of the object based on the capacitance pattern.
